(19) Europäisches Patentamt
European Patent Office
Office européen des brevets

(11) EP 3 040 406 B1

(12) **EUROPEAN PATENT SPECIFICATION**

(45) Date of publication and mention of the grant of the patent:
**25.04.2018 Bulletin 2018/17**

(21) Application number: **14833086.3**

(22) Date of filing: **17.07.2014**

(51) Int Cl.:
***C11B 5/00*** *(2006.01)* ***A23L 27/00*** *(2016.01)*
***A23L 27/20*** *(2016.01)* ***A61K 8/60*** *(2006.01)*
***A61Q 13/00*** *(2006.01)* ***C11B 9/00*** *(2006.01)*
***C11C 3/06*** *(2006.01)* ***A23L 2/60*** *(2006.01)*

(86) International application number:
**PCT/JP2014/069041**

(87) International publication number:
**WO 2015/016077 (05.02.2015 Gazette 2015/05)**

(54) **METHOD FOR STABILIZING SCENT COMPONENT, FRAGRANCE COMPOSITION, AND DEODORIZING COMPOSITION**

VERFAHREN ZUR STABILISIERUNG EINER DUFTKOMPONENTE, DUFTSTOFFZUSAMMENSETZUNG UND DESODORIERENDE ZUSAMMENSETZUNG

PROCÉDÉ POUR LA STABILISATION DE CONSTITUANT DE PARFUM, COMPOSITION DE FRAGRANCE ET COMPOSITION DÉSODORISANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2013 JP 2013159080**
**30.09.2013 JP 2013203832**

(43) Date of publication of application:
**06.07.2016 Bulletin 2016/27**

(73) Proprietor: **Toyo Sugar Refining Co., Ltd.**
**Tokyo 103-0016 (JP)**

(72) Inventors:
- **SATO, Shuichi**
**Ichihara-shi**
**Chiba 290-0046 (JP)**
- **AIZAWA, Yasushi**
**Ichihara-shi**
**Chiba 290-0046 (JP)**
- **IIDA, Yoshihisa**
**Tokyo 103-0016 (JP)**

(74) Representative: **Towler, Philip Dean**
**Dehns**
**St Bride's House**
**10 Salisbury Square**
**London EC4Y 8JD (GB)**

(56) References cited:
**EP-A1- 1 317 884**
**EP-A1- 1 541 660**
**JP-A- 2000 045 183**
**JP-A- 2004 229 668**
**JP-A- 2004 331 582**
**JP-A- 2007 137 862**
**JP-B2- 3 569 432**
**US-A1- 2010 158 840**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a method for preventing deterioration of smell components (fragrance components such as perfumes, and odor-causing components such as unsaturated fatty acids), and a perfume composition and a deodorant composition each of which utilizes this method. Further, the present invention also relates to uses of D-glucopyranosylglycerol.

### Background Art

In the past, perfumes have been used by adding them to food and drink and other products to which fragrances are intended to be imparted, in order to enhance qualities of those products. However, when the perfumes are subjected to actions of heat, light, air, enzyme, etc. not only in storage thereof but also in production process, storage process, distribution process and other processes subsequent to the addition of the perfumes, their fragrance and flavor components are deteriorated through an action of oxidation, reduction, hydrolysis, polymerization, volatilation or the like, thereby lowering the qualities of the products.

In particular, powder perfumes obtained by pulverization have an extremely large surface area, and therefore, deterioration takes place especially in the pulverization process or the storage. In emulsion perfumes obtained by emulsification, there occurs a problem that deterioration tends to take place in the emulsificationprocess or the storage due to the existence of water.

Then, a great number of proposals have been made to stabilize perfumes. For example, a method of utilizing a sequestering agent, flavonols, a sunflower extract and an apple extract (patent literature 1), a method of utilizing flavonols and neohesperidin dihydrochalcone (patent literature 2), a method of utilizing palatinose and raffinose (patent literature 3), a method of utilizing trehalose and water-soluble hemicellulose (patent literature 4), a method of utilizing cyclodextrin (patent literature 5), a method of utilizing 1, 5-anhydrofructose (patent literature 6), etc. can be mentioned.

By the methods described in the patent literatures 1 to 6, however, deterioration of perfumes cannot be sufficiently prevented in some cases.

On the other hand, there are deodorant products in the cosmetics group that has been now domestically paid attention. In particular, the elderly men who are nervous about aging odors and young women are eager for more effective products.

Most of causative substances of body odors are lower aldehydes or lower fatty acids, and secreted sebum is decomposed and oxidized by microorganisms, heat, light or the like to form these components. As a typical example, nonenal having an unpleasant oily odor, a so-called aging odor, can be mentioned, and this compound is a lower aldehyde formed by deterioration of palmitoleic acid that is a kind of unsaturated fatty acid contained in sebum. A means capable of suppressing formation of such lower aldehydes or lower fatty acids is being required.

By the way, α-D-glucopyranosylglycerol is contained in refined sake in an amount of about 0.5% by mass and is a substance that is known to impart refreshing sweetness. In recent years, it has been reported that the α-D-glucopyranosylglycerol has functions, such as non-cariogenic property, indigestible property and moisture retaining property, and usefulness of it as a functional material has been paid attention. Noting these functions, utilization of the α-D-glucopyranosylglycerol as a man component of seasonings or moisturizers and addition of it to a variety of food and drink and cosmetics have been proposed (patent literature 7).

Further, it has been also proposed that by the use of a saccharide composition containing α-D-glucopyranosylglycerol (α-D-dihydroxypropyl glucopyranoside) and its steric isomer, β-D-glucopyranosylglycerol (β-D-dihydroxypropyl glucopyranoside), in a specific ratio, moisture retaining property can be enhanced as compared with the case of using α-D-glucopyranosylglycerol alone (patent literature 8).

In the patent literatures 7 and 8, however, it is not described that α- and/or β-D-glucopyranosylglycerol can be utilized for preventing deterioration of fragrance components (perfumes, etc.) or odor-causing components (unsaturated fatty acids, etc.).

### Citation List

#### Patent Literature

Patent literature 1: JP H08(1996)-023940 A
Patent literature 2: JP 2000-236860 A

Patent literature 3: JP 2001-186858 A
Patent literature 4: JP 2000-217538 A
Patent literature 5: JP H11(1999)-222455 A
Patent literature 6: JP 2003-268397 A
Patent literature 7: JP H11(1999)-222496 A
Patent literature 8: JP 2011-236152 A

Summary of Invention

Technical Problem

The present invention addresses, in one aspect, the problem of providing a method capable of preventing deterioration (oxidation, decomposition or the like) of various smell components, e.g., fragrance components such as perfumes or odor-causing components such as unsaturated fatty acids. The present invention addresses, in another aspect, the problem of providing a perfume composition in which deterioration of a perfume has been prevented (a stabilized perfume composition) and a deodorant composition capable of preventing deterioration of an unsaturated fatty acid.

Solution to Problem

The present inventors have found that D-glucopyranosylglycerol has an action to prevent deterioration of a fragrance component such as a perfume or an odor-causing component such as an unsaturated fatty acid and can be utilized as an active ingredient of a stabilized perfume composition or a deodorant composition, and they have completed the present invention.

That is to say, the present invention includes the following matters.

[1] A method for preventing deterioration of a smell component, comprising allowing D-glucopyranosylglycerol to coexist with a smell component.
[2] The method for preventing deterioration as stated in [1], wherein the smell component is a perfume.
[3] The method for preventing deterioration as stated in [2], wherein the perfume is at least one perfume selected from the group consisting of citrus-based, fruity, floral, confectionery-based, ozone-based, marine-based, powdery, soapy, spicy, green-based, chypre-based, fougere-based, woody and aldehyde-based perfumes.
[4] The method for preventing deterioration as stated in [1], wherein the smell component is an unsaturated fatty acid.
[5] The method for preventing deterioration as stated in [1], wherein the D-glucopyranosylglycerol is a mixture of 1-O-$\alpha$-D-glucopyranosylglycerol,
1-O-$\beta$-D-glucopyranosylglycerol,
2-O-$\alpha$-D-glucopyranosylglycerol and
2-O-$\beta$-D-glucopyranosylglycerol.

Advantageous Effects of Invention

In the present invention, by utilizing D-glucopyranosylglycerol as an active ingredient for preventing deterioration of a fragrance component (perfume or the like) and an odor-causing component (unsaturated fatty acid or the like), a method for preventing deterioration of these components can be carried out. In addition, there can be produced high-quality products wherein problems regarding odors have been solved, solution of said problems having been so far demanded, such as a stabilized perfume composition capable of maintaining a fragrance and a flavor of a perfume for a long period of time and also capable of suppressing a deterioration odor and a deodorant composition capable of suppressing body odors such as an aging odor.

It is presumed that such mechanism as described below acts on the prevention of deterioration of a fragrance component and an odor-causing component by the D-glucopyranosylglycerol. That is to say, it is presumed that D-glucopyranosylglycerol having a structure in which saccharide has undergone addition to glycerol forms a molecular aggregate such as a micell or a cluster together with various fragrance components and odor-causing components, and it suppresses diffusion (motion) of those components or can cover a part that is susceptible to a change of a chemical structure, whereby deterioration of a perfume is suppressed to allow the fragrance to last, or oxidation or decomposition of an unsaturated bond part of a chemically unstable unsaturated fatty acid can be suppressed to prevent formation of an odor component. Contrary to this, as can be seen from comparison between the later-described examples and comparative examples, saccharides such as sucrose, anhydrofructose, sorbitol, D-glucose, erythritol, dextrin and glucose fructose liquid sugar, and glycerol are not found to have such excellent deterioration preventive actions on both of a

perfume and an unsaturated fatty acid as is possessed by the D-glucopyranosylglycerol, and it is presumed that such mechanism as above does not act on those compounds.

Description of Embodiments

In the present invention, the term "smell components" refers to smell-related components (compounds) in general and includes a "fragrance component" that stimulates the sense of smell to allow a pleasant fragrance to be perceptible and an "odor component" (including an "odor-causing component" that forms an odor component through deterioration such as decomposition) that allows an unpleasant odor to be perceptible. Typical examples of the fragrance components include various compounds generally used as "perfumes", and typical examples of the odor components include "unsaturated fatty acids" that causes body odors, etc.

The "method for preventing deterioration of a smell component" includes a method for retaining a pleasant fragrance by preventing deterioration of a fragrance component and a method for suppressing occurrence of an unpleasant odor by preventing deterioration of an odor component. The expression "preventing deterioration" of a fragrance component (perfume) can be put in another expression "stabilization" of the fragrance component. Such a method for preventing deterioration of a smell component can be carried out by allowing D-glucopyranosylglycerol to coexist with a smell component, that is, by bringing D-glucopyranosylglycerol and a fragrance component such as a perfume or an odor-causing component such as an unsaturated fatty acid into contact with each other in such a manner that their mutual interaction, which is presumed as previously described, takes place.

In the present description, a method relating to prevention of deterioration (stabilization) of a fragrance component, a product pertaining to performance of the method, or the like is referred to as a "first embodiment", and a method relating to prevention of deterioration of an odor-causing component, a product pertaining to performance of the method, or the like is referred to as a "second embodiment".

The working effect on prevention of deterioration of a fragrance component or an odor-causing component can be evaluated by a publicly known technique such as an organoleptic test. In the present invention, deterioration of a fragrance component or an odor-causing component does not always have to be prevented completely, and if it is shown from the results of an organoleptic test or the like that the degree of deterioration of a fragrance component or an odor-causing component is lower than usual (the case where the present invention is not carried out) or that deterioration of a fragrance component or an odor-causing component is prevented over a long period of time, this means that the working effect on prevention of deterioration of a fragrance component or an odor-causing component has been exerted, and it can be said that their embodiments correspond to the "method for preventing deterioration of a fragrance component" and the "stabilized perfume composition", or the "method for preventing deterioration of an odor-causing component" and the "deodorant composition".

- D-Glucopyranosylglycerol -

In the present invention, D-glucopyranosylglycerol is used as an essential substance for carrying out the method for preventing deterioration (for stabilization) of a fragrance component of the first embodiment and the method for preventing deterioration of an odor component of the second embodiment. In other words, the present invention provides uses of D-glucopyranosylglycerol as an active ingredient (deterioration inhibitor) for preventing deterioration of each of the fragrance component and the odor component.

D-Glucopyranosylglycerol is a general term for a compound represented by the formula (1), namely, 1-O-(α- or β-)D-(mono or poly)glucopyranosylglycerol (sometimes also referred to as "1-O-(α- or β-)D-dihydroxypropyl (mono or poly) glucopyranoside") and a compound represented by the formula (2), namely, 2-O-(α- or β-)D-(mono or poly)glucopyranosylglycerol (sometimes also referred to as "2-O-(α- or β-)D-dihydroxypropyl (mono or poly)glucopyranoside"). There is a structurally isomeric relationship between these compounds. In the present invention, only the compound represented by the formula (1) may be used, or only the compound represented by the formula (2) may be used, or a mixture of the compound represented by the formula (1) and the compound represented by the formula (2) may be used.

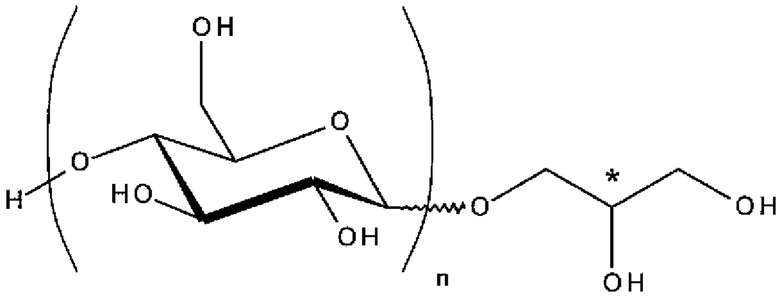

(1)

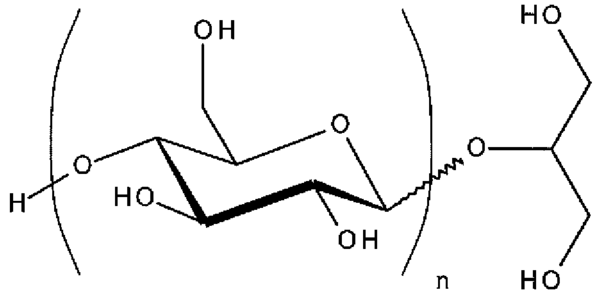

(2)

In the formula (1), the 2-position carbon atom with a symbol * is an asymmetric carbon atom, and optical isomers, namely, (2R) form and (2S) form, are present. The compound represented by the formula (1) may be any of these optical isomers, and may be a mixture (racemate) composed of these optical isomers.

In the formulas (1) and (2), n represents a degree of saccharide condensation and is an integer of 1 or more. With regard to a compound in a saccharide composition obtained by such a production process as described later, n is usually an integer of 1 to 6, mostly an integer of 1 to 4. In the present invention, only a compound in which n is 1 may be used, or a mixture of compounds in each of which n is 1 or more may be used.

In the formulas (1) and (2), the wavy line indicates that the 1-position carbon atom of a glucose residue in parentheses and an oxygen atom derived from a hydroxyl group of glycerol, and further, in case of n=2 or more, an oxygen atom derived from the 4-position hydroxyl group of a glucose residue and the 1-position carbon atom of a glucose reside, are linked by a single bond, and that the linkage may be an α-linkage or may be a β-linkage. In the present invention, only a compound in which the wavy line part is an α-linkage may be used, or only a compound in which the wavy line part is a β-linkage may be used, or a mixture of a compound in which the wavy line is an α-linkage and a compound in which the wavy line part is a β-linkage may be used.

Specific examples of the compounds represented by the formula (1) include a compound represented by the following formula (1A), said compound corresponding to a compound of the formula (1) in which n=1 and the wavy line represents an α-linkage, namely, 1-O-α-D-monoglucopyranosylglycerol, and a compound represented by the following formula (1B), said compound corresponding to a compound of the formula (1) in which n=1 and the wavy line represents a β-linkage, namely, 1-O-β-D-monoglucopyranosylglycerol. A specific example of the compound represented by the formula (2) is a compound represented by the following formula (2A), said compound corresponding to a compound of the formula (2) in which n=1 and the wavy line represents an α-linkage, namely, 2-O-α-D-monoglucopyranosylglycerol.

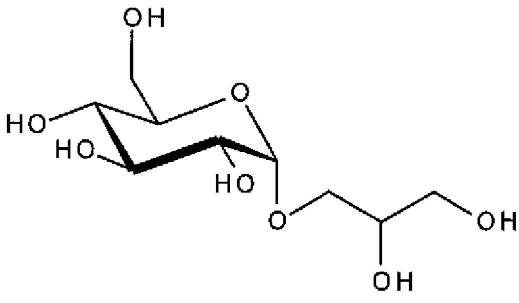

(1A)

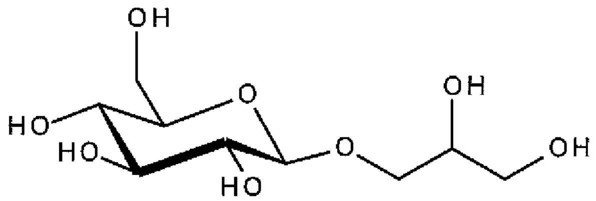

(1 B)

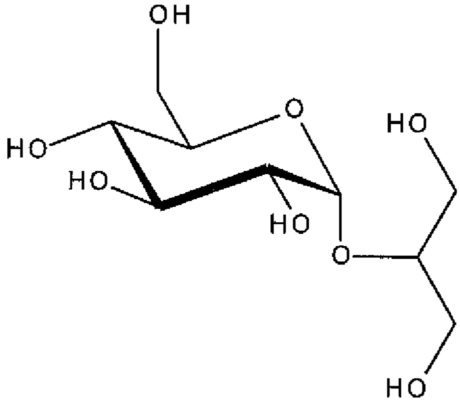

(2 A)

The form and the purity (content ratio) of the D-glucopyranosylglycerol for use in the present invention are not restricted. Examples of the forms of the D-glucopyranosylglycerol include an aqueous solution and a syrup-like high-viscosity solution obtained by concentrating an aqueous solution. The purity of the D-glucopyranosylglycerol is preferably not less than 10% by weight, and for example, the purity of the D-glucopyranosylglycerol in the high-viscosity solution can be set to about 40% by weight or more.

As a component other than the D-glucopyranosylglycerol, there can be mentioned, for example, an unreacted material or a reaction by-product (impurity), which is sometimes introduced when D-glucopyranosylglycerol is prepared by an organic synthesis method.

Examples of such impurities include glycerol that is added as one raw material for synthesizing D-glucopyranosylglycerol and reaction by-products derived from glycerol, e.g., alcohols, such as ethanol, isopropyl alcohol, ethylene glycol, diethylene glycol, triethylene glycol, propylene glycol, dipropylene glycol, 1,3-butylene glycol, 1,2-pentanediol, diglycerol and polyglycerol.

The amount of glycerol compounded in food and drink, seasonings, cosmetics, etc. is sometimes restricted. On that account, when the perfume composition according to the first embodiment of the present invention or the deodorant composition according to the second embodiment of the present invention is used for producing those products, the amount of glycerol in a saccharide composition (composition containing D-glucopyranosylglycerol and the aforesaid unre acted material and reaction by-product) obtained by the organic synthesis method is preferably not more than 20% by mass, more preferably not more than 10% by mass, based on the total solids content in the saccharide composition. On the other hand, when the amount of the glycerol compounded is not particularly a problem, the amount of glycerol may be not less than 20% by mass based on the total solids content in the saccharide composition.

Further, as the aforesaid impurities, a glucose source that is added as the other raw material for synthesizing D-glucopyranosylglycerol and reaction by-products derived from the glucose source, such as saccharide formed by decomposition of di- or higher saccharide when the glucose source is the di- or higher saccharide, and saccharide produced as a by-product through the reaction of such saccharides, can be mentioned.

D-Glucopyranosylglycerol can be prepared in advance by a publicly known technique, or can be purchased as a commercial product.

For example, if such an enzyme process as described in JP H11(1999)-0222496 A (patent literature 7) is used, a product containing α-D-glucopyranosylglycerol as a main component is obtained, and if such an organic synthesis method as described in JP 2011-236152 A (patent literature 8) is used, a mixture containing α-D-glucopyranosylglycerol and β-D-glucopyranosylglycerol in a given ratio is obtained. The outline of the organic synthesis method is described below. This production process comprises a step of allowing a glucose source to react with glycerol using an acid catalyst to synthesize D-glucopyranosylglycerol (glycosylationstep) and a step of adding a neutralizing agent for the acid catalyst into the reaction system of the glycosilation step to terminate the reaction (reaction termination step), and if necessary, may further comprise a refining step (distillation step, decoloring step or the like) for removing residual glycerol, a residual

glucose source, a by-product, etc., an aqueous solution preparation step of lowering the viscosity to enhance handling properties, and other steps.

For example, a commercial product "COSARTE-2G" manufactured by Toyo Sugar Refining Co., Ltd. is a composition containing D-glucopyranosylglycerol (mixture of
1-O-α-D-glucopyranosylglycerol,
1-O-β-D-glucopyranosylglycerol,
2-O-α-D-glucopyranosylglycerol and
2-O-β-D-glucopyranosylglycerol), water and glycerol, and the content of D-dihydroxypropyl glucopyranoside, excluding water and glycerol, is about 70 to 78% and is 73.5% on an average. When the method for preventing deterioration of a smell component according to the present invention is carried out or when the perfume composition or the deodorant composition according to the present invention is produced, it is preferable to use, as the D-glucopyranosylglycerol, the above commercial product containing the above four kinds of compounds.

- Method for preventing deterioration of fragrance component

In the first embodiment of the method for preventing deterioration of a smell component according to the present invention, the object of the deterioration prevention is a fragrance component, typically a perfume, as the smell component. In this embodiment, D-glucopyranosylglycerol can be used in the same mode as that for a publicly known stabilizer for perfumes. That is to say, D-glucopyranosylglycerol can be used for stabilizing a fragrance component (perfume) by allowing D-glucopyranosylglycerol to coexist with a fragrance component that is intended to be prevented from deterioration, generally by adding it to a perfume, a product containing a perfume, or the like.

Specifically, the method for preventing deterioration of a perfume according to the first embodiment may be carried out by adding D-glucopyranosylglycerol to a perfume in the production of such a perfume composition as described later, or may be carried out by adding D-glucopyranosylglycerol to a perfume and other components in the production of a product (composition) using a perfume. Further, if a perfume and D-glucopyranosylglycerol are in a coexisting state after production of a perfume composition or a product containing a perfume, it can be said that the above method has been carried out.

Also in the case where a previously prepared perfume composition containing a perfume and D-glucopyranosylglycerol is not added but a perfume and D-glucopyranosylglycerol are separately added in the production of a product (composition), the content of D-glucopyranosylglycerol in the product is not restricted provided that it is in a range where the working effect on prevention of deterioration of the perfume is exerted. The deterioration prevention effect can be exhibited by adding D-glucopyranosylglycerol generally in an amount of 100 times or more, preferably 1,000 times or more, the amount by mass of the perfume added and used in the product. In this case, the upper limit of the amount of D-glucopyranosylglycerol added is not specif ically restricted, but the upper limit is properly determined taking influence on the properties of the product into consideration.

• Perfume

The type and the shape of the perfume that is a typical fragrance component are not restricted. The perfumes may be natural perfumes, such as essential oil, extract, oleoresin, recovered flavor and isolated perfume, or may be synthetic perfumes, such as alcohols, esters, aldehydes, acetals and lactones. The perfume may be any one of them or may comprise two or more kinds of them (compound perfume material).

When perfumes are classified by the general system in the technical field of perfumes, there can be mentioned the following as specific examples of the perfumes: citrus-basedperfumes (lemon, lime, grapefruit, etc.), fruity perfumes (orange, apple, melon, grape, pineapple, banana, peach, strawberry, raspberry, mango, passion fruit, mandarin, apricot, black currant, etc.), floral perfumes (rose, jasmine, lavender, lily bell, magnolia, violet, neroli, lilac, freesia, muguet, gardenia, etc.), confectionery-based perfumes (vanilla, etc.), ozone-based perfumes (hyacinth, magnolia, iris, etc.), marine-based perfumes (water mint, water lily, water fruit, fresh pepper, ginger, etc.), powderyperfumes (violet, cyclamen, Tonkabean, heliotrope, etc.), soapy perfumes (musk, etc.), spicy perfumes (bergamot, pepper, cinnamon, nutmeg, clove, etc.), green-based perfumes (geranium, sage, eucalyptus, mint, chamomile, lavender, rosemary, etc.), chypre-based perfumes (oakmoss, bergamot, vetiver, fern, etc.), fougere-basedperfumes (lavender, etc.), woody perfumes (cypress, sandalwood, cedar wood, patchouli, vetiver, ivy leaf, etc.) and aldehyde-based perfumes (aldehyde C9, aldehyde C10, aldehyde C11, aldehyde C12, etc.). Examples of the shapes of the perfumes include powder, emulsion, paste and solution.

- Perfume composition -

The perfume composition that is described herein contains at least one perfume and D-glucopyranosylglycerol as an active ingredient for preventing deterioration of the perfume. However, the D-glucopyranosylglycerol is not hindered

from further exertion of other functions in the perfume composition or a product containing the perfume composition. The perfume composition according to the present invention may be a composition essntially consisting of D-glucopyranosylglycerol and at least one perfume only, or maybe a composition containing D-glucopyranosylglycerol, at least one perfume and other arbitrary components, as long as the perfume composition exerts a working effect on stabilization of the perfume.

The content of the D-glucopyranosylglycerol in the perfume composition is not restricted provided that it is in a range where the working effect on prevention of deterioration of the perfume is exerted. Such a content can be properly determined by a person skilled in the art according to the perfume used, without requiring excess trials and error, while examining a relationship between the content of the D-glucopyranosylglycerol and the perfume deterioration prevention effect.

The deterioration prevention effect can be exhibited by adding D-glucopyranosylglycerol generally in an amount of 0.1 time or more, preferably 1 time or more, the amount by mass of the perfume used. In this case, the upper limit of the amount of D-glucopyranosylglycerol added is not specifically restricted, but the upper limit is properly determined taking handling properties, cost, etc. of the perfume composition into consideration.

When a commercial product also containing other components is used in order to incorporate D-glucopyranosylglycerol, the above description of the content of D-glucopyranosylglycerol in the perfume composition refers to not a content based on the mass of the commercial product itself but a content based on the mass of D-glucopyranosylglycerol (calculable from the mass of the commercial product and the purity of D-glucopyranosylglycerol) contained in the commercial product.

The perfume composition described herein may contain the following substances (compounds) according to the dosage form, when needed: emulsifying agents, such as glycerol fatty acid ester, sorbitan fatty acid ester, sucrose fatty acid ester, lecithin, and enzyme-treated lecithin; thickening polysaccharides (viscosity modifiers), such as guar gum, xanthan gum, tamarind seed gum, pectin, agar, carrageenan, arabinoxylan, arabinogalactan, water-soluble hemicellulose, and gum arabic; organic acids, such as lactic acid, citric acid, malic acid, acetic acid, carbonic acid and phosphoric acid, and salts thereof, such as sodium salt, potassium salt, calcium salt and magnesium salt; oils and fats, ethanol, water, propylene glycol, starch and chemically modified starches, dextrin, casein; antioxidants (oxidation inhibitors, reducing agents), such as vitamin C, glutachione, glutachione-containing yeast extract, cysteine, cystine, vitamin E, vitamin C fatty acid ester, tea catechin and polyphenol; and publicly known various components used for cosmetics, quasi-drugs, foods, etc.

Although the production process for the perfume composition is not restricted, the perfume composition can be produced by adding D-glucopyranosylglycerol to at least one perfume in accordance with a conventional method. The perfume may be prepared in advance by a publicly known technique, or may be purchased as a commercial product.

The method to add D-glucopyranosylglycerol to the perfume is not restricted. For example, when the perfume is liquid, a method of dissolving D-glucopyranosylglycerol in the solution can be mentioned. When the perfume is a powder, a method comprising dissolving the powder perfume once, then adding D-glucopyranosylglycerol to the solution and drying, a method of allowing the powder perfume to adsorb liquid D-glucopyranosylglycerol, a method comprising further drying the adsorption product, etc. can be mentioned. When the perfume is in a state of emulsion, a method of adding D-glucopyranosylglycerol to the emulsion can be mentioned. Further, a method of directly adding or kneading the perfume into a highly concentrated solution of D-glucopyranosylglycerol can be also mentioned.

The apparatus and the technique to carry out such a production process for the perfume composition as above are not restricted. For example, when a liquid or paste-like perfume composition is produced, a stirrer, a kneader or the like can be used; when a powdery perfume composition is produced, a drying method, such as spray drying method, hot air drying method, vacuum freeze drying method, vacuum drying method or drum drying method, and an apparatus for the method can be used; and when an emulsion-like perfume composition is produced, a homomixer, a homogenizer, a colloid mill or the like can be used.

The perfume composition described herein produced may be stored inside a closed vessel or the like until a product containing the perfume composition is produced, or it can be immediately used for producing a product containing the perfume composition. When the perfume composition is stored inside a closed vessel or the like, it is preferable to store it in such an environment that the perfume composition is influenced by light, heat, air or the like, which causes deterioration of the perfume, as little as possible.

The perfume composition described herein can be used in the production of various products (compositions) by compounding it as a part of the components of each product (composition). The product in which the perfume composition is to be compounded is not restricted, and a variety of products required to be scented, such as cosmetics, quasi-drugs, other daily life products (oil, cream, lipstick, lotion, shampoo, conditioner, soap, hand soap, mouthwash, aromatic, aroma candle, etc.), and food and drink (beverage, frozen sweet, dessert, confectionery, retort pouched food, instant food, seasoning, etc.), can be mentioned.

Such products containing the perfume composition can be produced by the same process as a conventional process, except that its step is changed in such a manner that the perfume composition described herein is compounded

instead of a part or all of a perfume or a perfume composition compounded in a conventional product.

- Method for preventing deterioration of odor-causing component -

In the second embodiment of the method for preventing deterioration of a smell component according to the present invention, the object of the deterioration prevention is an odor-causing component, typically an unsaturated fatty acid, as the smell component. In this embodiment, D-glucopyranosylglycerol can be used in the same mode as that for a publicly known deodorant or odor-eliminating component. That is to say, D-glucopyranosylglycerol can be used in order to prevent deterioration of an odor component and thereby inhibit occurrence of an unpleasant odor by applying D-glucopyranosylglycerol to an odor component (causative component) that is intended to be prevented from deterioration, generally by adding D-glucopyranosylglycerol as a deodorant component to a deodorant product or the like and applying the product to a body part that emits a body odor.

• Unsaturated fatty acid

The unsaturated fatty acid that is a typical odor-causing component is not specifically restricted provided that it is decomposed and oxidized by microorganism, heat, light or the like to form a lower aldehyde (nonenal or the like) or a lower fatty acid, which becomes a body odor. Examples of such unsaturated fatty acids include oleic acid, linoleic acid, linolenic acid, palmitoleic acid and vaccenic acid.

- Deodorant composition -

The deodorant composition that is provided as one aspect of the second embodiment of the present invention contains D-glucopyranosylglycerol as an active ingredient for preventing deterioration of an unsaturated fatty acid, and may further contain other arbitrary components. However, the D-glucopyranosylglycerol is not hindered from further exertion of other functions in the deodorant composition.

The content of the D-glucopyranosylglycerol in the deodorant composition is not restricted provided that it is in a range where the working effect on suppression of a body odor is exerted when the deodorant composition is applied to the body surface. Such a content can be properly determined by a person skilled in the art according to the dosage form of the deodorant composition through an appropriate evaluation test, without requiring excess trials and errors. For example, when the deodorant composition is a deodorant agent, the content of the D-glucopyranosylglycerol is generally 0.01 to 50% by weight, preferably 0.5 to 10% by weight, based on the total weight of the deodorant composition.

The deodorant composition can be generally prepared as a product (composition) whose main purpose is to prevent a body odor or the like, or as a product (composition) applied to a body surface of a human or other animal whose main purpose is a matter other than the prevention of a body odor or the like but to which a function to prevent a body odor or the like is intended to be further added. Such products may be cosmetics, quasi-drugs or other daily life products, and examples thereof include deodorant products and care products for hair, etc. (aerosol, cream, lotion, spray, stick, shampoo, conditioner, treatment, hair tonic, body soap, etc.).

Such a deodorant composition (product) according to the second embodiment as above can be produced by the same process as a conventional process, except that its step is changed in such a manner that the D-glucopyranosylglycerol is compounded instead of a part or all of a deodorant component compounded in a conventional product.

The deodorant composition may contain the following substances (compounds) according to the dosage form, when needed: odor-eliminating powders (chitosan fine particles, amphoteric porous fine particles, zeolite, antibacterial zeolite, porous silica, zinc oxide, magnesium oxide, etc.); bactericides (3,4,4-trichlorocarbanilide, triclosan, benzalkonium chloride, benzethonium chloride, alkyltrimethylammonium chloride, resorcin, phenol, sorbic acid, salicylic acid, hexachlorophen, isopropyl methylphenol, silver ion, copper ion, zinc ion, etc.); polyphenols (phloroglucinol; fluoroglucinol derivatives such as aspidin and aspidinol; tannin; tannin derivatives such as pyrogallol tannin and catechol tannin; plant extracts containing polyphenols, such as mimosa, green tea, mint, senna and horse chestnut); antiperspirants (aluminum chlorohydrate, aluminum zirconium chlorohydrate, aluminum chloride, aluminum sulfate, basic aluminum bromide, aluminumphenolsulfonic acid, basic aluminum iodide, etc.); others, such as publicly known various components used in cosmetics, quasi-drugs, foods, etc.

Examples

[Example 1] Citrus-based perfume

To 1,000 g of water, 300 g of gum arabic, 80 g of D-glucopyranosylglycerol (COSARTE-2G, manufactured by Toyo Sugar RefiningCo., Ltd.) and 130 g of dextrin (DE10) were added to dissolve them, and the resulting solution was

subjected to heat sterilization at 85 to 90°C for 15 minutes. After the solution was cooled to 40°C, 70 g of lemon oil was added, and they were emulsified by an ultrasonic homogenizer and spray-dried by a spray dryer to obtain a powder lemon perfume (Example 1). Further, by using sucrose instead of D-glucopyranosylglycerol, a powder lemon perfume was obtained similarly to the above (Comparative Example 1). Each of the powder lemon perfumes was placed in a plastic bag, then each bag was sealed, and each of the perfumes was stored at 40°C for 3 months. Thereafter, 0.02 g of each of the powder lemon perfumes was dissolved in 100 g of water, and organoleptic tests were carried out by 7 panelists. The results are set forth in Table 1. With regard to fragrance, flavor and deterioration odor, 10-grade evaluation was carried out. The most intensive emission was taken as 10, and no emission was taken as 0. The numerical value was a mean value.

Table 1

| | Fragrance | Flavor | Deterioration odor |
|---|---|---|---|
| Ex. 1 | 9 | 9 | 0 |
| Comp. Ex. 1 | 3 | 3 | 8 |

[Example 2] Fruity perfume

In 300 g of water, 30 g of sucrose fatty acid ester (HLB16) and 300 g of D-glucopyranosylglycerol (COSARTE-2G, manufactured by Toyo Sugar Refining Co. , Ltd.) were dissolved, and the resulting solution was subjected to heat sterilization at 65 to 75°C for 30 minutes. After the solution was cooled to 40°C, 50 g of orange oil was added, and they were emulsified by an ultrasonic homogenizer and cooled down to 25°C to obtain an orange emulsion perfume (Example 2). Further, by using glycerol instead of D-glucopyranosylglycerol, an orange emulsion perfume was obtained similarly to the above (Comparative Example 2). Each of the orange emulsion perfumes was placed in a glass bottle, then each bottle was stoppered tightly, and each of the perfumes was stored at 40°C for 3 months. Thereafter, 0.03 g of each of the orange emulsion perfumes was dissolved in 100 g of water, and organoleptic tests were carried out by 7 panelists. The results are set forth in Table 2. With regard to fragrance, flavor and deterioration odor, 10-grade evaluation was carried out. The most intensive emission was taken as 10, and no emission was taken as 0. The numerical value was a mean value.

Table 2

| | Fragrance | Flavor | Deterioration odor |
|---|---|---|---|
| Ex. 2 | 9 | 9 | 1 |
| Comp. Ex. 2 | 3 | 3.5 | 8 |

[Example 3] Floral perfume

To 500 g of a 50% (W/W%) ethanol aqueous solution, 160 g of maltosylcyclodextrin and 130 g of D-glucopyranosylglycerol (COSARTE-2G, manufactured by Toyo Sugar Refining Co., Ltd.) were added, and theywere dissolved by heating at 60 to 65°C. Thereafter, 40 g of rose oil was introduced into the resulting solution, and they were emulsified by an ultrasonic homogenizer and cooled down to 25°C to obtain a rose emulsion perfume (Example 3). Further, by using anhydrofructose (Anhydrose, manufactured by Nihon Starch Co., Ltd.) instead of D-glucopyranosylglycerol, a rose emulsion perfume was obtained similarly to the above (Comparative Example 3). Each of the rose emulsion perfumes was placed in a glass bottle, then each bottle was stoppered tightly, and each of the perfumes was stored at 40°C for 3 months. Thereafter, 0.03 g of each of the rose emulsion perfumes was dissolved in 100 g of water, and organoleptic tests were carried out by 7 panelists. The results are set forth in Table 3. With regard to fragrance, flavor and deterioration odor, 10-graze evaluation was carried out. The most intensive emission was taken as 10, and no emission was taken as 0. The numerical value was a mean value.

Table 3

| | Fragrance | Flavor | Deterioration odor |
|---|---|---|---|
| Ex. 3 | 9 | 9 | 1 |
| Comp. Ex. 3 | 7.5 | 7 | 3 |

[Example 4] Confectionery-based perfume

To 500 g of a 50% (W/W%) ethanol aqueous solution, 150 g of D-glucopyranosylglycerol (COSARTE-2G, manufactured by Toyo Sugar Refining Co., Ltd.) was added, and they were mixed. Thereafter, 80 g of a vanilla flavor was added to the resulting mixture, and they were stirred for 5 minutes by a stirrer to obtain a vanilla perfume formulation (Example 4). Further, by using glycerol instead of D-glucopyranosylglycerol, a vanilla perfume formulation was obtained similarly to the above (Comparative Example 4). Each of the vanilla perfume formulations was placed in a glass bottle, then each bottle was stoppered tightly, and each of the formulations was storedat 40°C for 3 months . Thereafter, 0.03 g of each of the vanilla perfume formulations was dissolved in 100 g of water, and organoleptic tests were carried out by 7 panelists. The results are set forth in Table 4. With regard to fragrance, flavor and deterioration odor, 10-grade evaluation was carried out. The most intensive emission was taken as 10, and no emission was taken as 0. The numerical value was a mean value.

Table 4

| | Fragrance | Flavor | Deterioration odor |
|---|---|---|---|
| Ex. 4 | 9 | 9 | 1 |
| Comp. Ex. 4 | 5 | 5 | 4 |

[Example 5] Ozone-based perfume

To 500 g of a 50% (W/W%) ethanol aqueous solution, 120 g of D-glucopyranosylglycerol (COSARTE-2G, manufactured by Toyo Sugar Refining Co., Ltd.) was added, and they were mixed. Thereafter, 70 g of an ozone-based flavor was added to the resulting mixture, and they were stirred for 5 minutes by a stirrer to obtain an ozone perfume formulation (Example 5). Further, by using sorbitol instead of D-glucopyranosylglycerol, an ozone perfume formulation was obtained similarly to the above (Comparative Example 5). Each of the ozone perfume formulations was placed in a glass bottle, then each bottle was stoppered tightly, and each of the formulations was storedat 40 ° C for 3 months. Thereafter, 0.03 g of each of the ozone perfume formulations was dissolved in 100 g of water, and organoleptic tests were carried out by 7 panelists. The results are set forth in Table 5. With regard to fragrance and deterioration odor, 10-grade evaluation was carried out. The most intensive emission was taken as 10, and no emission was taken as 0. The numerical value was a mean value.

Table 5

| | Fragrance | Deterioration odor |
|---|---|---|
| Ex. 5 | 9 | 1 |
| Comp. Ex. 5 | 7 | 3 |

[Example 6] Marine-based perfume

To 500 g of a 50% (W/W%) ethanol aqueous solution, 160 g of maltosylcyclodextrin and 130 g of D-glucopyranosylglycerol (COSARTE-2G, manufactured by Toyo Sugar Refining Co. , Ltd.) were added, and they were dissolved by heating at 60 to 65°C. Thereafter, 40 g of water mint oil was added to the resulting solution, and they were emulsified by an ultrasonic homogenizer and cooled down to 25 ° C to obtain a water mint emulsionperfume (Example 6) . Further, by using D-gulcose instead of D-glucopyranosylglycerol, a water mint emulsion perfume was obtained similarly to the above (Comparative Example 6). Each of the water mint emulsion perfumes was placed in a glass bottle, then each bottle was stoppered tightly, and each of the perfumes was stored at 40 °C for 3 months. Thereafter, 0.03 g of each of the water mint emulsion perfumes was dissolved in 100 g of water, and organoleptic tests were carried out by 7 panelists. The results are set forth in Table 6. With regard to fragrance, flavor and deterioration odor, 10-grade evaluation was carried out. The most intensive emission was taken as 10, and no emission was taken as 0. The numerical value was a mean value.

Table 6

| | Fragrance | Flavor | Deterioration odor |
|---|---|---|---|
| Ex. 6 | 9 | 9 | 0 |

(continued)

| | Fragrance | Flavor | Deterioration odor |
|---|---|---|---|
| Comp. Ex. 6 | 5 | 4 | 2 |

[Example 7] Powdery perfume

To 1,000 g of water, 300 g of gum arabic, 80 g of D-glucopyranosylglycerol (COSARTE-2G, manufactured by Toyo Sugar Refining Co., Ltd.), 130 g of dextrin (DE10) and 80 g of a violet powder were added to dissolve them, and the resulting solution was subjected to heat sterilization at 70 to 75 °C for 30 minutes. After the solution was cooled to 40°C, it was emulsified by an ultrasonic homogenizer and spray-dried by a spray dryer to obtain a powder violet perfume (Example 7). Further, by using glycerol instead of D-glucopyranosylglycerol, a powder violet perfume was obtained similarly to the above (Comparative Example 7). Each of the powder violet perfumes was placed in a plastic bag, then each bag was sealed, and each of the perfumes was stored at 40°C for 3 months. Thereafter, 0.02 g of each of the powder violet perfumes was dissolved in 100 g of water, and organoleptic tests were carried out by 7 panelists. The results are set forth in Table 7 . With regard to fragrance, flavor and deterioration odor, 10-grade evaluation was carried out. The most intensive emission was taken as 10, and no emission was taken as 0. The numerical value was a mean value.

Table 7

| | Fragrance | Flavor | Deterioration odor |
|---|---|---|---|
| Ex. 7 | 9 | 9 | 0 |
| Comp. Ex. 7 | 4 | 4 | 3 |

[Example 8] Soapy perfume

To 500 g of a 50% (W/W%) ethanol aqueous solution, 150 g of D-glucopyranosylglycerol (COSARTE-2G, manufactured by Toyo Sugar Refining Co., Ltd.) was added, and they were mixed. Thereafter, 70 g of a musk flavor was added to the resulting mixture, and they were stirred for 5 minutes by a stirrer to obtain a musk perfume formulation (Example 8). Further, by using erythritol instead of D-glucopyranosylglycerol, a musk perfume formulation was obtained similarly to the above (Comparative Example 8). Each of the musk perfume formulations was placed in a glass bottle, then each bottle was stoppered tightly, and each of the formulations was stored at 40°C for 3 months. Thereafter, 0.03 g of each of the musk perfume formulations was dissolved in 100 g of water, and organoleptic tests were carried out by 7 panelists . The results are set forth in Table 8. With regard to fragrance and deterioration odor, 10-grade evaluation was carried out. The most intensive emission was taken as 10, and no emission was taken as 0. The numerical value was a mean value.

Table 8

| | Fragrance | Deterioration odor |
|---|---|---|
| Ex. 8 | 9 | 1 |
| Comp. Ex. 8 | 4 | 3 |

[Example 9] Spicy perfume

To 1,000 g of water, 300 g of gum arabic, 100 g of D-glucopyranosylglycerol (COSARTE-2G, manufactured by Toyo Sugar Refining Co., Ltd.) and 150 g of dextrin (DE10) were added to dissolve them, and the resulting solution was subjected to heat sterilization at 70 to 75°C for 30 minutes. After the solution was cooled to 40 °C, 20 g of bergamot oil was added, and they were emulsified by an ultrasonic homogenizer and spray-dried by a spray dryer to obtain a powder bergamot perfume (Example 9). Further, by using glycerol instead of D-glucopyranosylglycerol, a powder bergamot perfume was obtained similarly to the above (Comparative Example 9). Each of the powder bergamot perfumes was placed in a plastic bag, then each bag was sealed, and each of the perfumes was stored at 40°C for 3 months. Thereafter, 0.02 g of each of the powder bergamot perfumes was dissolved in 100 g of water, and organoleptic tests were carried out by 7 panelists. The results are set forth in Table 9. With regard to fragrance, flavor and deterioration odor, 10-graze evaluation was carried out. The most intensive emission was taken as 10, and no emission was taken

as 0. The numerical value was a mean value.

Table 9

| | Fragrance | Flavor | Deterioration odor |
|---|---|---|---|
| Ex. 9 | 9 | 9 | 0 |
| Comp. Ex. 9 | 5.5 | 4 | 4 |

[Example 10] Green-based perfume

To 500 g of a 50% (W/W%) ethanol aqueous solution, 140 g of D-glucopyranosylglycerol (COSARTE-2G, manufactured by Toyo Sugar Refining Co., Ltd.) was added, and they were mixed. Thereafter, 60 g of a geranium flavor was added to the resulting mixture, and they were stirred for 10 minutes by a stirrer to obtain a geranium perfume formulation (Example 10). Further, by using anhydrofructose (Anhydrose, manufactured by Nihon Starch Co., Ltd.) instead of D-glucopyranosylglycerol, a geranium perfume formulation was obtained similarly to the above (Comparative Example 10). Each of the geranium perfume formulations was placed in a glass bottle, then each bottle was stoppered tightly, and each of the formulations was stored at 40°C for 3 months. Thereafter, 0.03 g of each of the geranium perfume formulations was dissolved in 100 g of water, and organoleptic tests were carried out by 7 panelists. The results are set forth in Table 10. With regard to fragrance and deterioration odor, 10-grade evaluation was carried out. The most intensive emission was taken as 10, and no emission was taken as 0. The numerical value was a mean value.

Table 10

| | Fragrance | Deterioration odor |
|---|---|---|
| Ex. 10 | 9 | 1 |
| Comp. Ex. 10 | 6 | 3 |

[Example 11] Chypre-based perfume

To 500 g of a 50% (W/W%) ethanol aqueous solution, 120 g of D-glucopyranosylglycerol (COSARTE-2G, manufactured by Toyo Sugar Refining Co., Ltd.) was added, and they were mixed. Thereafter, 60 g of an oakmoss flavor was added to the resulting mixture, and they were stirred for 5 minutes by a stirrer to obtain an oakmoss perfume formulation (Example 11). Further, by using dextrin instead of D-glucopyranosylglycerol, an oakmoss perfume formulation was obtained similarly to the above (Comparative Example 11). Each of the oakmoss perfume formulations was placed in a glass bottle, then each bottle was stoppered tightly, and each of the formulations was storedat 40 ° C for 3 months . Thereafter, 0.03 g of each of the oakmoss perfume formulations was dissolved in 100 g of water, and organoleptic tests were carried out by 7 panelists. The results are set forth in Table 11. With regard to fragrance and deterioration odor, 10-grade evaluation was carried out. The most intensive emission was taken as 10, and no emission was taken as 0. The numerical value was a mean value.

Table 11

| | Fragrance | Deterioration odor |
|---|---|---|
| Ex. 11 | 9 | 1 |
| Comp. Ex. 11 | 7.5 | 2 |

[Example 12] Fougere-based perfume

To 500 g of a 50% (W/W%) ethanol aqueous solution, 100 g of D-glucopyranosylglycerol (COSARTE-2G, manufactured by Toyo Sugar Refining Co., Ltd.) was added, and they were mixed. Thereafter, 40 g of lavender oil was added to the resulting mixture, and they were stirred for 5 minutes by a stirrer to obtain a lavender perfume formulation (Example 12). Further, by using glucose fructose liquid sugar instead of D-glucopyranosylglycerol, a lavender perfume formulation was obtained similarly to the above (Comparative Example 12). Each of the lavender perfume formulations was placed in a glass bottle, then each bottle was stoppered tightly, and each of the formulations was stored at 40 °C for 3 months. Thereafter, 0.03 g of each of the lavender perfume formulations was dissolved in 100 g of water, and

organoleptic tests were carried out by 7 panelists. The results are set forth in Table 12. With regard to fragrance and deterioration odor, 10-grade evaluation was carried out. The most intensive emission was taken as 10, and no emission was taken as 0. The numerical value was a mean value.

Table 12

| | Fragrance | Deterioration odor |
|---|---|---|
| Ex. 12 | 9 | 1 |
| Comp. Ex. 12 | 6 | 3 |

[Example 13] Woody perfume

To 500 g of a 50% (W/W%) ethanol aqueous solution, 100 g of D-glucopyranosylglycerol (COSARTE-2G, manufactured by Toyo Sugar Refining Co., Ltd.) was added, and they were mixed. Thereafter, 40 g of a cypress flavor was added to the resulting mixture, and they were stirred for 5 minutes by a stirrer to obtain a cypress perfume formulation (Example 13). Further, by using glycerol instead of D-glucopyranosylglycerol, a cypress perfume formulation was obtained similarly to the above (Comparative Example 13). Each of the cypress perfume formulations was placed in a glass bottle, then each bottle was stoppered tightly, and each of the formulations was storedat 40 ° C for 3 months . Thereafter, 0.03 g of each of the cypress perfume formulations was dissolved in 100 g of water, and organoleptic tests were carried out by 7 panelists. The results are set forth in Table 13. With regard to fragrance and deterioration odor, 10-grade evaluation was carried out. The most intensive emission was taken as 10, and no emission was taken as 0. The numerical value was a mean value.

Table 13

| | Fragrance | Deterioration odor |
|---|---|---|
| Ex. 13 | 9 | 1 |
| Comp. Ex. 13 | 5.5 | 2 |

[Example 14] Aldehyde-based perfume

To 500 g of a 50% (W/W%) ethanol aqueous solution, 100 g of D-glucopyranosylglycerol (COSARTE-2G, manufactured by Toyo Sugar Refining Co., Ltd.) was added, and they were mixed. Thereafter, 20 g of artificial aldehyde was introduced into the resulting mixture, and they were stirred for 5 minutes by a stirrer to obtain an aldehyde perfume formulation (Example 14). Further, by using glycerol instead of D-glucopyranosylglycerol, an aldehyde perfume formulation was obtained similarly to the above (Comparative Example 14). Each of the aldehyde perfume formulations was placed in a glass bottle, then each bottle was stoppered tightly, and each of the formulations was stored at 40 °C for 3 months. Thereafter, 0.03 g of each of the aldehyde perfume formulations was dissolved in 100 g of water, and organoleptic tests were carried out by 7 panelists. The results are set forth in Table 14. With regard to fragrance and deterioration odor, 10-grade evaluation was carried out. The most intensive emission was taken as 10, and no emission was taken as 0. The numerical value was a mean value.

Table 14

| | Fragrance | Deterioration odor |
|---|---|---|
| Ex. 14 | 9 | 0 |
| Comp. Ex. 14 | 5 | 2 |

[Example 15] Shampoo (1)

In a container equipped with a stirring device, about 50 ml of water, 16 g of sodium lauryl sulfate and 3.0 g of cocoyl betaine were placed, and they were stirred and dissolved. Next, 0.2 g of hydroxypropyl cellulose, 3 g of D-glucopyranosylglycerol (COSARTE-2G, manufactured by Toyo Sugar Refining Co., Ltd.), 0.003 g of a lemon perfume and an antiseptic were added, and they were stirred and mixed. After the resulting mixture was adjusted to pH 6.5 by the use of citric acid, water was added to make up a total amount of 100 g, whereby a lemon perfume shampoo was

obtained (Example 15). Further, by using glycerol instead of D-glucopyranosylglycerol, a lemon perfume shampoo was obtained similarly to the above (Comparative Example 15) . Each of the lemon perfume shampoos was placed in a glass bottle, then each bottle was stoppered tightly, and each of the shampoos was stored at 40°C for 5 months. Thereafter, in a 200 ml Erlenmeyer flask, 5 g of each of the lemon perfume shampoos and 50 g of water were placed, then each flask was stoppered tightly, and the contents in each flask were shaken and mixed for 30 seconds. Thereafter, organoleptic tests were carried out by 6 panelists. The results are set forth in Table 15. With regard to fragrance and deterioration odor, 10-grade evaluation was carried out. The most intensive emission was taken as 10, and no emission was taken as 0. The numerical value was a mean value.

Table 15

| | Fragrance | Deterioration odor |
|---|---|---|
| Ex. 15 | 9 | 1 |
| Comp. Ex. 15 | 6 | 4 |

[Example 16] Shampoo (2)

In a container equipped with a stirring device, about 50 ml of water, 10.0 g of an amino-modified polysiloxane emulsion, 5.0 g of 2-alkyl-N-carboxymethyl-N-hydroxyimidazolium betaine, 10 g of triethanolamine lauryl sulfate, 5 g of D-glucopyranosylglycerol (COSARTE-2G, manufactured by Toyo Sugar Refining Co., Ltd.), 0.005 g of a rose perfume and an antiseptic were placed, then they were stirred and mixed, and water was added to make up a total amount of 100 g, whereby a rose perfume shampoo was obtained (Example 16). Further, by using anhydrofructose (Anhydrose, manufactured by Nihon Starch Co., Ltd.) instead of D-glucopyranosylglycerol, a rose perfume shampoo was obtained similarly to the above (Comparative Example 16). Each of the rose perfume shampoos was placed in a glass bottle, then each bottle was stoppered tightly, and each of the shampoos was stored at 40°C for 5 months. Thereafter, in a 200 ml Erlenmeyer flask, 5 g of each of the rose perfume shampoos and 50 g of water were placed, then each flask was stoppered tightly, and the contents in each flask were shaken and mixed for 30 seconds. Thereafter, organoleptic tests were carried out by 8 panelists. The results are set forth in Table 16. With regard to fragrance and deterioration odor, 10-grade evaluation was carried out. The most intensive emission was taken as 10, and no emission was taken as 0. The numerical value was a mean value.

Table 16

| | Fragrance | Deterioration odor |
|---|---|---|
| Ex. 16 | 9 | 0 |
| Comp. Ex. 16 | 7 | 2 |

[Example 17] Body care oil

By the following formulation, body care oil containing D-glucopyranosylglycerol was prepared in a conventional way, and cyclomethicone was added to make up a total amount of 100 g (Example 17). Further, by using glycerol instead of D-glucopyranosylglycerol, similar body car oil was obtained (Comparative Example 17). Each of the body care oils was placed in a glass bottle, then each bottle was stoppered tightly, and each of the oils was stored at 40°C for 5 months. Thereafter, the glass bottles were opened, and organoleptic tests were carried out by 8 panelists. The results are set forth in Table 17. With regard to fragrance and deterioration odor, 10-grade evaluation was carried out. The most intensive emission was taken as 10, and no emission was taken as 0. The numerical value was a mean value.

| | |
|---|---|
| Caprylic/capric triglyceride | 7 g |
| Propylene glycol dicaprylate/dicaprate | 22 g |
| Cetostearyl octanoate, isopropyl myristate | 5 g |
| Isostearyl pivalate | 3 g |
| Groundnut oil | 1 g |
| Water mint oil | 0.003 g |
| D-Glucopyranosylglycerol or glycerol 5 | g |
| VE, antiseptic | proper amount |

Table 17

| | Fragrance | Deterioration odor |
|---|---|---|
| Ex. 17 | 9 | 0 |
| Comp. Ex. 17 | 7 | 2 |

[Example 18] Face care cream

By the following formulation, face care cream containing D-glucopyranosylglycerol was prepared in a conventional way, and water was added to make up a total amount of 100 g (Example 18). Further, by using glycerol instead of D-glucopyranosylglycerol, similar face care cream was obtained (Comparative Example 18). Each of the face care creams was placed in a glass bottle, then each bottle was stoppered tightly, and each of the creams was stored at 40°C for 5 months. Thereafter, the glass bottles were opened, and organoleptic tests were carried out by 8 panelists. The results are set forth in Table 18. With regard to fragrance and deterioration odor, 10-grade evaluation was carried out. The most intensive emission was taken as 10, and no emission was taken as 0. The numerical value was a mean value.

| | |
|---|---|
| Vaseline | 4 g |
| Hydrogenated polyisobutene | 7 g |
| Cetyl alcohol | 3 g |
| Sorbitan tristearate | 1 g |
| PEG40 stearate | 3 g |
| Myristyl myristate | 3 g |
| Glyceryl stearate | 3 g |
| Shea butter | 2 g |
| Cyclomethicone | 5 g |
| Stearic acid | 0.2 g |
| NaOH | 0.5 g |
| Citric acid soda | 0.1 g |
| Lavender perfume | 0.004 g |
| D-Glucopyranosylglycerol or glycerol | 5 g |
| VE, antiseptic | proper amount |

Table 18

| | Fragrance | Deterioration odor |
|---|---|---|
| Ex. 18 | 9 | 0 |
| Comp. Ex. 18 | 6.5 | 3 |

[Example 19] Lotion

By the following formulation, a lotion containing D-glucopyranosylglycerol was prepared in a conventional way, and water was added to make up a total amount of 100 g (Example 19). Further, by using propylene glycol (PG) instead of D-glucopyranosylglycerol, a similar lotion was obtained (Comparative Example 19). Each of the lotions was placed in a glass bottle, then each bottle was stoppered tightly, and each of the lotions was stored at 40 °C for 5 months. Thereafter, the glass bottles were opened, and organoleptic tests were carried out by 8 panelists. The results are set forth in Table 19. With regard to fragrance and deterioration odor, 10-grade evaluation was carried out. The most intensive emission was taken as 10, and no emission was taken as 0. The numerical value was a mean value.

| | |
|---|---|
| Hydrogenated egg yolk lecithin | 0.5 g |
| Liquid paraffin | 0.5 g |
| Polyoxyethylene(20) sorbitan monooleate | 0.5 g |
| Ethyl alcohol | 10 g |

(continued)

| | |
|---|---|
| Glycerol | 4 g |
| Jasmine flavor | 0.004 g |
| D-Glucopyranosylglycerol or PG | 5 g |
| Methylparaben | 0.1 g |

Table 19

| | Fragrance | Deterioration odor |
|---|---|---|
| Ex. 19 | 9 | 0 |
| Comp. Ex. 19 | 7 | 4 |

[Example 20] Oral wetting agent (gel type)

By the following formulation, an oral wetting agent (gel type) containing D-glucopyranosylglycerol was prepared in a conventional way, and water was added to make up a total amount of 100 g (Example 20). Further, by using sorbitol instead of D-glucopyranosylglycerol, a similar oral wetting agent (gel type) was obtained (Comparative Example 20). Each of the oral wetting agents was placed in a glass bottle, then each bottle was stoppered tightly, and each of the agents was stored at 40°C for 5 months. Thereafter, the glass bottles were opened, and organoleptic tests were carried out by 8 panelists. The results are set forth in Table 20. With regard to fragrance and deterioration odor, 10-grade evaluation was carried out. The most intensive emission was taken as 10, and no emission was taken as 0. The numerical value was a mean value.

| | |
|---|---|
| Carboxymethyl cellulose sodium | 4 g |
| Glycerol | 15 g |
| Propylene glycol | 5 g |
| Sodium benzoate | 0.3 g |
| Cetylpyridium chloride | 0.01 g |
| Citric acid | 0.23 g |
| Sodium citrate | 1.1 g |
| Spearmint perfume | 0.003 g |
| D-Glucopyranosylglycerol or sorbitol | 5 g |

Table 20

| | Fragrance | Deterioration odor |
|---|---|---|
| Ex. 20 | 9 | 0 |
| Comp. Ex. 20 | 6 | 2 |

[Example 21] Beverage

By the following formulation, a beverage containing D-glucopyranosylglycerol was prepared in a conventional way, and water was added to make up a total amount of 200 g (Example 21). Further, by using trehalose instead of D-glucopyranosylglycerol, a similar beverage was obtained (Comparative Example 21). Each of the beverages was placed in a glass bottle, then each bottle was stoppered tightly, and each of the beverages was stored at 40°C for 3 months. Thereafter, organoleptic tests were carried out by 8 panelists. The results are set forth in Table 21. With regard to fragrance, flavor and deterioration odor, 10-grade evaluation was carried out. The most intensive emission was taken as 10, and no emission was taken as 0. The numerical value was a mean value.

| | |
|---|---|
| Sugar | 10 g |
| Enzyme-treated stevia | 0.1 g |

(continued)

| | |
|---|---|
| Enzyme-treated hesperidin | 0.6 g |
| Orange concentrated juice | 2 g |
| Orange flavor | 0.01 g |
| D-Glucopyranosylglycerol or trehalose | 5 g |
| Acidulant | 1 g |
| Calcium lactate | 0.7 g |
| Vitamin C | 0.03 g |
| Vitamin $D_3$ | 150 IU |
| Vitamin K | 100 μg |

Table 21

| | Fragrance | Flavor | Deterioration odor |
|---|---|---|---|
| Ex. 21 | 9 | 9 | 0 |
| Comp. Ex. 21 | 7 | 6 | 2 |

[Examples 22 to 24] Suppression of deterioration odor of unsaturated fatty acid (organoleptic evaluation)

The following substances 1 to 8 containing unsaturated fatty acids (oleic acid, linoleic acid, linolenic acid) were weighed into a vial tube according to the composition shown in Tables 22 to 24, and they were mixed. The resulting mixture was heated in boiling water for 1 hour and cooled to room temperature to obtain a test sample. In Table 22, Example 22 and Comparative Examples 23 and 24 were compared with Comparative Example 22; in Table 23, Example 23 and Comparative Examples 26 and 27 were compared with Comparative Example 25; and in Table 24, Example 24 and Comparative Examples 29 and 30 were compared with Comparative Example 28. Evaluation was carried out by 8 panelists based on the following criteria.

<Substances used>

- Substance 1: hydroxyethyl cellulose (HEC SE-900, Daicel FineChem Ltd.) 0.5 g
- Substance 2: 0.6M phosphoric acid buffer solution (phosphoric acid (Nacalai Tesque, Inc.), dipotassium hydrogenphosphate (Kanto Chemical Co., Ltd.)) 0.25 ml
- Substance 3: oleic acid (Wako Pure Chemical Industries, Ltd.) 0.1 g
- Substance 4: linoleic acid (Kanto Chemical Co., Ltd.) 0.1 g
- Substance 5 : linonenic acid (Wako Pure Chemical Industries, Ltd.) 0.1 g
- Substance 6: 5% glyceryl glucoside aqueous solution (COSARTE-2G, Toyo Sugar Refining Co., Ltd.) 1.0 ml
- Substance 7: 5% glycerol aqueous solution (concentrated glycerol for cosmetics, Kao Corporation) 1.0 ml
- Substance 8: 5% glucose aqueous solution (D-(+)-glucose, Kanto Chemical Co., Ltd.) 1.0 ml

<Evaluation criteria>

-2: The test sample hardly smells.
-1: The test sample does not smell much.
0: The smell is nearly equal.
1: The test sample smells a little.
2: The test sample considerably smells.

Table 22

| Composition (amount added) | Ex. | Comp. Ex. | | |
|---|---|---|---|---|
| | 22 | 22 | 23 | 24 |
| Hydroxyethyl cellulose (g) | 0.5 | 0.5 | 0.5 | 0.5 |
| 0.6M Phosphoric acid buffer solution (ml) | 0.25 | 0.25 | 0.25 | 0.25 |
| Oleic acid (g) | 0.1 | 0.1 | 0.1 | 0.1 |
| Purified water | - | 1.0 | - | - |
| 5% Glyceryl glucoside aqueous solution (ml) | 1.0 | - | - | - |
| 5% Glucose aqueous solution (ml) | - | - | 0.1 | - |
| 5% Glycerol aqueous solution (ml) | - | - | - | 0.1 |
| Panelist evaluation (point(s)) | -5 | 0 | 4 | 7 |

Table 23

| Composition (amount added) | Ex. | Comp. Ex. | | |
|---|---|---|---|---|
| | 23 | 25 | 26 | 27 |
| Hydroxyethyl cellulose (g) | 0.5 | 0.5 | 0.5 | 0.5 |
| 0.6M Phosphoric acid buffer solution (ml) | 0.25 | 0.25 | 0.25 | 0.25 |
| Linoleic acid (g) | 0.1 | 0.1 | 0.1 | 0.1 |
| Purified water | - | 1.0 | - | - |
| 5% Glyceryl glucoside aqueous solution (ml) | 1.0 | - | - | - |
| 5% Glucose aqueous solution (ml) | - | - | 0.1 | - |
| 5% Glycerol aqueous solution (ml) | - | - | - | 0.1 |
| Panelist evaluation (point(s)) | -2 | 0 | 4 | 8 |

Table 24

| Composition (amount added) | Ex. | Comp. Ex. | | |
|---|---|---|---|---|
| | 24 | 28 | 29 | 30 |
| Hydroxyethyl cellulose (g) | 0.5 | 0.5 | 0.5 | 0.5 |
| 0.6M Phosphoric acid buffer solution (ml) | 0.25 | 0.25 | 0.25 | 0.25 |
| Linolenic acid (g) | 0.1 | 0.1 | 0.1 | 0.1 |
| Purified water | - | 1.0 | - | - |
| 5% Glyceryl glucoside aqueous solution (ml) | 1.0 | - | - | - |
| 5% Glucose aqueous solution (ml) | - | - | 0.1 | - |
| 5% Glycerol aqueous solution (ml) | - | - | - | 0.1 |
| Panelist evaluation (point(s)) | -5 | 0 | 2 | 4 |

As a result of the above organoleptic evaluation, it can be seen that the deterioration odors of the unsaturated fatty acids of oleic acid, linoleic acid and linolenic acid were reduced by glyceryl glucoside.

[Example 25] Effect on suppression of deterioration odor of linolenic acid (gas chromatography analysis)

Similarly to Examples 22 to 24, the following substances 1, 2 and 5 to 9 containing linolenic acid were weighed into a vial tube according to the composition shown in Table 25, and they were mixed. The resulting mixture was heated in boiling water for 1 hour, then cooled to room temperature and thereafter heated at 80°C for 5 minutes in a hot water bath to obtain a test sample. The test sample was subjected to gas chromatography analysis under the following conditions, and using peak area values of four kinds of fatty acid deterioration odor components (1) to (4), an occurrence ratio of the odor component was calculated from the following formula. As a control, Comparative Example 31 was used, and as the test samples, Example 25 and Comparative Examples 32 to 34 in Table 25 were used.

$$\text{Odor component occurrence ratio (\%)} = \left( \frac{\text{Test sample peak area value}}{\text{Control peak area value}} \right) \times 100$$

<Substances used>

- Substance 1: hydroxyethyl cellulose (HEC SE-900, Daicel FineChem Ltd.) 0.5 g
- Substance 2: 0.6M phosphoric acid buffer solution (phosphoric acid (Nacalai Tesque, Inc.), dipotassium hydrogenphosphate (Kanto Chemical Co., Ltd.)) 0.25 ml
- Substance 5 : linolenic acid (Wako Pure Chemical Industries, Ltd.) 0.1 g
- Substance 6: 5% glyceryl glucoside aqueous solution (COSARTE-2G, Toyo Sugar Refining Co., Ltd.) 1.0 ml
- Substance 7: 5% glycerol aqueous solution (concentrated glycerol for cosmetics, Kao Corporation) 1.0 ml
- Substance 8: 5% glucose aqueous solution (D-(+)-glucose, Kanto Chemical Co., Ltd.) 1.0 ml
- Substance 9: 5% trehalose aqueous solution (trehalose, Hayashibara Biochemical Laboratories, Inc.) 1.0 ml

<Gas chromatography analytical conditions>

Type of equipment: Gas chromatograph Shimadzu GC-2010
Preparation: solid phase microextraction method, 10 minutes
Elimination: 5 minutes
Injection mode: splitless
Temperature of constant-temperature bath: 40°C (1 minute) → 100°C, 5°C/1 minute
Carrier gas: He 72.0 cm/SEC
Detector: MS

Table 25

| Composition (amount added) | Ex. | Comp. Ex. | | | |
|---|---|---|---|---|---|
| | 25 | 31 | 32 | 33 | 34 |
| Hydroxyethyl cellulose (g) | 0.5 | 0.5 | 0.5 | 0.5 | 0.5 |
| 0.6M Phosphoric acid buffer solution (ml) | 0.25 | 0.25 | 0.25 | 0.25 | 0.25 |
| Linolenic acid (g) | 0.1 | 0.1 | 0.1 | 0.1 | 0.1 |
| Purified water | - | 1.0 | - | - | - |
| 5% Glyceryl glucoside aqueous solution (ml) | 1.0 | - | - | - | - |
| 5% Glucose aqueous solution (ml) | - | - | 1.0 | - | - |
| 5% Glycerol aqueous solution (ml) | - | - | - | 1.0 | - |

(continued)

| Composition (amount added) | Ex. | Comp. Ex. | | | |
|---|---|---|---|---|---|
| | 25 | 31 | 32 | 33 | 34 |
| 5% Trehalose aqueous solution (ml) | - | - | - | - | 1.0 |
| Fatty acid deterioration odor component (i) occurrence ratio (%) | 60 | 100 | 108 | 108 | 124 |
| Fatty acid deterioration odor component (ii) occurrence ratio (%) | 67 | 100 | 119 | 101 | 133 |
| Fatty acid deterioration odor component (iii) occurrence ratio (%) | 71 | 100 | 106 | 154 | 95 |
| Fatty acid deterioration odor component (iv) occurrence ratio (%) | 64 | 100 | 122 | 122 | 83 |

As a result of the above gas chromatography analysis, it can be seen that four kinds of the deterioration odors generated by heating linolenic acid were reduced by the addition of glyceryl glucoside. From the above, it has been understood that glyceryl glucoside is excellent in the effect on suppression of deterioration odors of fatty acids, and is a useful substance applicable also to cosmetics, etc.

## Claims

1. A method for preventing deterioration of a smell component, comprising allowing D-glucopyranosylglycerol to coexist with a smell component.

2. The method for preventing deterioration as claimed in claim 1, wherein the smell component is a perfume.

3. The method for preventing deterioration as claimed in claim 2, wherein the perfume is at least one perfume selected from the group consisting of citrus-based, fruity, floral, confectionery-based, ozone-based, marine-based, powdery, soapy, spicy, green-based, chypre-based, fougere-based, woody and aldehyde-based perfumes.

4. The method for preventing deterioration as claimed in claim 1, wherein the smell component is an unsaturated fatty acid.

5. The method for preventing deterioration as claimed in claim 1, wherein the D-glucopyranosylglycerol is a mixture of 1-O-α-D-glucopyranosylglycerol, 1-O-β-D-glucopyranosylglycerol, 2-O-α-D-glucopyranosylglycerol and 2-O-β-D-glucopyranosylglycerol.

## Patentansprüche

1. Verfahren zu dem Verhindern einer Verschlechterung einer Geruchskomponente, umfassend das Koexistierenlassen von D-Glucopyranosylglycerol mit einer Geruchskomponente.

2. Verfahren zu dem Verhindern einer Verschlechterung nach Anspruch 1, wobei es sich bei der Geruchskomponente um ein Parfüm handelt.

3. Verfahren zu dem Verhindern einer Verschlechterung nach Anspruch 2, wobei es sich bei dem Parfüm um mindestens ein Parfüm handelt, das aus der Gruppe bestehend aus citrusbasierten, fruchtigen, blumigen, süßwarenbasierten, ozonbasierten, meerwasserbasierten, pudrigen, seifigen, würzigen, grünpflanzenbasierten, chyprebasierten, fougerebasierten, holzigen und aldehydbasierten Parfüms ausgewählt ist.

4. Verfahren zu dem Verhindern einer Verschlechterung nach Anspruch 1, wobei die Geruchskomponente eine ungesättigte Fettsäure ist.

5. Verfahren zu dem Verhindern einer Verschlechterung nach Anspruch 1, wobei es sich bei dem D-Glucopyranosylglycerol um eine Mischung aus 1-O-α-D-Glucopyranosylglycerol, 1-O-β-D-Glucopyranosylglycerol, 2-O-α-D-Glucopyranosylglycerol und 2-O-β-D-Glucopyranosylglycerol handelt.

## Revendications

1. Procédé pour empêcher la détérioration d'un composant d'odeur, comprenant le fait de permettre à du D-glucopyranosylglycérol de coexister avec un composant d'odeur.

2. Procédé pour empêcher la détérioration selon la revendication 1, dans lequel le composant d'odeur est un parfum.

3. Procédé pour empêcher la détérioration selon la revendication 2, dans lequel le parfum est au moins un parfum choisi dans le groupe constitué de parfums à base d'agrumes, fruités, floraux, à base de confiserie, à base d'ozone, à base marine, pulvérulents, savonneux, épicés, à base de végétaux, à base de parfum de chypre, à base de fougère, boisés et à base d'aldéhyde.

4. Procédé pour empêcher la détérioration selon la revendication 1, dans lequel le composant d'odeur est un acide gras insaturé.

5. Procédé pour empêcher la détérioration selon la revendication 1, dans lequel le D-glucopyranosylglycérol est un mélange de 1-O-α-D-glucopyranosylglycérol, de 1-O-β-D-glucopyranosylglycérol, de 2-O-α-D-glucopyranosylglycérol et de 2-O-β-D-glucopyranosylglycérol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H081996023940 A
- JP 2000236860 A
- JP 2001186858 A
- JP 2000217538 A
- JP H111999222455 A
- JP 2003268397 A
- JP H111999222496 A
- JP 2011236152 A
- JP H1119990222496 A